(19) Europäisches Patentamt / European Patent Office / Office européen des brevets

(11) **EP 1 036 179 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**01.01.2014   Patentblatt 2014/01**

(45) Hinweis auf die Patenterteilung:
**09.11.2005   Patentblatt 2005/45**

(21) Anmeldenummer: **98965223.5**

(22) Anmeldetag: **02.12.1998**

(51) Int Cl.:
**C12N 15/12** *(2006.01)*      **C07K 14/505** *(2006.01)*
**C12P 21/00** *(2006.01)*      **C12N 5/00** *(2006.01)*

(86) Internationale Anmeldenummer:
**PCT/EP1998/007819**

(87) Internationale Veröffentlichungsnummer:
**WO 1999/028455 (10.06.1999 Gazette 1999/23)**

(54) **VERFAHREN ZUR HERSTELLUNG VON POLYPEPTIDEN MIT GEEIGNETER GLYKOSILIERUNG**

METHOD FOR PREPARING POLYPEPTIDES WITH APPROPRIATE GLYCOSILATION

PROCEDE DE PREPARATION DE POLYPEPTIDES A GLYCOSYLATION APPROPRIEE

(84) Benannte Vertragsstaaten:
**AT BE CH DE DK ES FI FR GB GR IE IT LI LU NL PT SE**

(30) Priorität: **03.12.1997   DE 19753681**
**17.07.1998   EP 98113409**

(43) Veröffentlichungstag der Anmeldung:
**20.09.2000   Patentblatt 2000/38**

(73) Patentinhaber: **Roche Diagnostics GmbH**
**68305 Mannheim (DE)**

(72) Erfinder:
• **FRANZE, Reinhard**
**D-82377 Penzberg (DE)**
• **EBERHARDT, Horst**
**D-37083 Göttingen (DE)**
• **WALLERIUS, Claus**
**D-82377 Penzberg (DE)**

(74) Vertreter: **Weiss, Wolfgang et al**
**Weickmann & Weickmann**
**Patentanwälte**
**Postfach 86 08 20**
**81635 München (DE)**

(56) Entgegenhaltungen:
**US-A- 4 935 350**

• **DATABASE WPI Section Ch, Week 9502 Derwent Publications Ltd., London, GB; Class B04, AN 95-009082 XP002085768 & JP 06 292592 A (SNOW BRAND MILK PROD CO LTD), 21. Oktober 1994 in der Anmeldung erwähnt**

• **YODA K. ET AL.: "Time course of glutamine in a culture medium during high-density culture of mouse C127 transformants" ANNALS OF THE NEW YORK ACADEMY OF SCIENCES, Bd. 750, 1995, Seiten 175-179, XP002085765**

• **PANNEERSELVAM K. ET AL.: "Human fibroblasts prefer Mannose over Glucose as a source of Mannose for N-glycosylation" THE JOURNAL OF BIOLOGICAL CHEMISTRY, Bd. 272, Nr. 37, 12. September 1997, Seiten 23123-23129, XP002085766**

• **LJUNGGREN J. UND HÄGGSTRÖM L.: "Catabolic control of hybridoma cells by Glucose and Glutamine limited fed batch cultures" BIOTECHNOLOGY AND BIOENGINEERING, Bd. 44, Nr. 7, 20. September 1994, Seiten 808-818, XP002007048**

• **ANDERSEN D.C. UND GOOCHEE C.F.: "The effect of cell-culture conditions on the oligosaccharide structures of secreted glycoproteins" CURRENT OPINION IN BIOTECHNOLOGY, Bd. 5, 1994, Seiten 546-549, XP002085767**

• **TAKEUCHI M. ET AL.: "Relatioship between sugar chain structure and biological activity of recombinant human Erythropoietin produced in Chinese Hamster Ovary cells" PROCEEDINGS OF THE NATIONAL ACADEMY OF SCIENCES OF USA, Bd. 86, Oktober 1989, Seiten 7819-7822, XP002053701 in der Anmeldung erwähnt**

• **XIE L. UND WANG D.I.C.: "Integrated approaches to the design of media and feeding strategies for fed-batch cultures of animal cells" TRENDS IN BIOTECHNOLOGY, Bd. 15, Nr. 3, März 1997, Seite 109-113 XP004054783**

EP 1 036 179 B2

**Beschreibung**

Die Erfindung betrifft ein Verfahren zur Erhöhung des Glykosilierungsgrades bei der Herstellung eines Polypeptids durch Kultivierung von menschlichen Zellen und die Gewinnung des Polypeptids aus dem Kulturmedium oder/und den Zellen. Das gewünschte glykosilierte Polypeptid kann dabei rekombinant, mit Hilfe endogener Genaktivierung oder natürlicherweise von den Zellen produziert werden.

Die Herstellung von Glykoproteinen durch Kultivierung eukaryontischer Zellen erfolgt im allgemeinen in handelsüblichen Kulturmedien. Dabei kann der Zusatz bestimmter Substrate zu dem Kulturmedium notwendig sein, um eine gewünschte Glykosilierung des Polypeptids zu erreichen. Dies ist für ein Batch-Verfahren in kleinen Volumina (< 1000 ml), einer niedrigen Startzelldichte ($5 \times 10^4$ Zellen/ml) und einer kurzen Kultivierungsdauer (48 h) beispielhaft an einem humanen IgM-Antikörper in der japanischen Offenlegungsschrift H6-292 592 beschrieben. Dort wird bei der rekombinanten Herstellung von Antikörpern in Säugerzellen, z.B. CHO-Zellen, anstelle der üblicherweise verwendeten Glucose ein anderer Zucker, beispielsweise Fructose, Mannose, Galactose, N-Acetylglucosamin, Ribose, Fucose, N-Acetylgalactosamin oder dergleichen verwendet. Weiterhin wird ein mehrstufiges Kultivierungsverfahren offenbart, bei dem die Zellen zunächst in einem glucosehaltigen Medium kultiviert werden, welches anschließend durch ein einen anderen Zucker enthaltendes Medium ausgetauscht wird.

Das in der japanischen Offenlegungsschrift H6-29592 beschriebene Verfahren hat jedoch schwerwiegende Nachteile. Durch den Verbrauch des Zuckers während der Zellkultivierung verändert sich ständig dessen Konzentration im Kulturmedium, so daß ein gleichbleibend hoher Glykosilierungsgrad der Polypeptide nicht gewährleistet ist. Das Batch-Verfahren ist außerdem ungeeignet, wenn für eine gewünschte Glykosilierung eine gleichbleibende Substratkonzentration erforderlich ist, da sich die Anfangskonzentrationen der Substrate durch den Zellstoffwechsel ständig verringern. Weiterhin müssen die für hohe Zelldichten und einen gleichbleibend hohen Glykosilierungsgrad erforderlichen hohen Zuckerkonzentrationen bereits zu Anfang der Fermentation vorgelegt werden, wodurch jedoch das Wachstum der Zellen gehemmt und somit die erreichbare Zelldichte begrenzt wird. Eine wirtschaftliche Produktion hochglykosilierter Polypeptide mit dem in der oben genannten japanischen Offenlegungsschrift beschriebenen Verfahren ist daher nicht möglich.

Die Kultivierung eukaryontischer Zellen durch ein Batch-Verfahren mit Zufütterung (Fed-Batch), bei dem während der Kultivierung Nährlösung zugegeben wird, ist bekannt. Bei dieser Art von Verfahren kann durch geeignete Zufütterung eine höhere Zelldichte und eine längere Kultivierungsdauer erzielt werden. In diesem Zusammenhang ist die Veröffentlichung von Ljunggren und Häggström (Biotech. Bioeng. 44 (1994), 808-818) zu nennen, in welcher der Einfluß von Substrat-limitierten Zufütterungskulturen bei der Gewinnung eines Antikörpers aus Mauszellen beschrieben wird. Die Zellen werden hierbei unter kontrollierter Zugabe von Glucose und Glutamin gezüchtet. Die Autoren fanden eine Reduktion von unerwünschten Nebenprodukten und einen positiven Effekt auf die Antikörperproduktion durch die niedrigen Konzentrationen von Glucose und Glutamin. Ein weiteres Beispiel ist die kontinuierliche und limitierte Zufütterung der essentiellen Aminosäure Glutamin, die zu einem verbesserten Zellwachstum führt (Ljunggren et al. Biotech. Lett. 12 (1990), 705-710). Die Zielsetzung der Zufütterung von Glutamin ist die Reduzierung der Ammoniumbildung, da Ammonium toxisch für tierische Zellen ist (Mirabet et al., Biotechnol. Bioeng. 56 (1997), 530-537).

Gawlitzek et al. (Biotechnol. Bioeng. 57 (1998), 518-528) beschreiben, daß durch erhöhte Konzentrationen von Ammoniumionen oder Glucosamin im Medium von kultivierten eukaryontischen BHK-21 Zellen eine Zunahme der Komplexität von N-gebundenen Kohlenhydratstrukturen in rekombinanten Glykoproteinen gefunden wird, die von den kultivierten Zellen sekretiert werden. Dieser Befund steht jedoch im Widerspruch zu Ergebnissen von Borys et al. (Biotechnol. Bioeng. 43 (1994), 505-514) oder Andersen und Goochee (Biotechnol. Bioeng. 47 (1995), 96-105), wo eine Hemmung der Glykosilierung durch erhöhte Ammoniumkonzentrationen im Kulturmedium festgestellt wurden. Somit wird deutlich, daß die Kontrolle der Ammoniumbildung in der Kultur nur ein isolierter Aspekt und daher nicht ausreichend für die wirtschaftliche Herstellung von Proteinen mit geeigneter Glykosilierung ist.

Der Glykosilierungsgrad von Polypeptiden kann einen großen Einfluß auf deren biologische Aktivität haben. Dies wird im folgenden Beispiel von Erythropoietin (EPO) erläutert. EPO ist ein humanes Glycoprotein, welches die Produktion von roten Blutzellen stimuliert. EPO kommt im Blutplasma von gesunden Personen nur in sehr geringen Konzentrationen vor, so daß eine Bereitstellung in größeren Mengen auf diese Weise nicht möglich ist. EP-B-0 148 605 und EP-B-0 205 564 beschreiben die Herstellung von rekombinantem humanen EPO in CHO-Zellen. Das in EP-B-0 148 605 beschriebene EPO weist ein höheres Molekulargewicht als urinäres EPO und keine O-Glykosilierung auf. Das in EP-B-0 205 564 beschriebene EPO aus CHO-Zellen ist mittlerweile in großen Mengen und in reiner Form verfügbar.

Weiterhin ist die Gewinnung von humanem EPO aus dem Urin von Patienten mit aplastischer Anämie bekannt (Miyake et al., J. Biol. Chem. 252 (1977), 5558-5564).

Rekombinantes und urinäres EPO wird als Gemisch verschiedener Isoformen gewonnen, von denen bekannt ist, daß sie sich in ihrem Sialylierungsgrad unterscheiden. Diese EPO-Isoformen weisen unterschiedliche isoelektrische Punkte auf und können durch isoelektrische Fokussierung bzw. Kapillarelektrophorese aufgetrennt werden (siehe Tsao et al., Biotech. Bioeng. 40 (1992), 1190-1196; Nieto et al., Anal. Commun. 33 (1996), 425-427; Tran et al., J. Chromatogr.

542 (1991), 459-471; Bietot et al., J. Chromatogr. 759 (1997), 177-184; Watson et al. Anal. Biochem. 210 (1993), 389-393). Die Isoformen mit der höchsten Anzahl von Sialinsäuren weisen die höchste spezifische Aktivität auf, während die mit der niedrigsten Anzahl die geringste Aktivität besitzen (siehe z.B. Imai et al., Eur. J. Biochem. 194 (1990), 457-462; EP-A-0 428 267).

Takeuchi et al., (Proc. Natl. Acad. Sci. USA 86 (1989), 7819-7822) beschreiben einen Zusammenhang der biologischen Aktivität mit dem Sialinsäuregehalt und dem Verhältnis von bi- und tetraantennären Kohlenhydratstrukturen. Takeuchi et al. kommen weiterhin zur Schlußfolgerung, daß die in den EPO-Kohlenhydratstrukturen vorhandenen N-Acetyl-Lactosamin-Disaccharideinheiten nicht mit der biologischen Aktivität korrelieren.

Fukuda et al. (Blood 73 (1989), 84-89) befassen sich mit der Geschwindigkeit der Elimination von EPO aus dem Blutkreislauf, die wesentlich zur biologischen Aktivität beiträgt, und kommen zu dem Schluß, daß EPO mit einer größeren Anzahl an N-Acetyl-Lactosamin-Einheiten schneller aus dem Kreislauf entfernt wird als EPO ohne Lactosamin-Einheiten. Morimoto et al. (Glycoconjugate J. 13 (1996), 1093-1120) beschreiben die Auftrennung von EPO-Isoformen mittels Mono-Q-Chromatographie, so daß die einzelnen Fraktionen jeweils aus nur noch wenigen Isoformen bestehen. Die mit diesen Fraktionen durchgeführten Untersuchungen zeigen eine Gleichverteilung aller Strukturen in allen Fraktionen. Es wird keine Korrelation des Gehalts an bi- oder triantennären Strukturen oder des Gehalts an N-Acetyl-Lactosamin-Einheiten mit der spezifischen Aktivität gefunden.

Somit geht aus dem genannten Stand der Technik hervor, daßeine generelle Korrelation der biologischen Aktivität mit der Zuckerstruktur besteht, insbesondere im Hinblick auf den Gehalt an Sialinsäuren.

Überraschenderweise wurde festgestellt, daß durch eine kontinuierliche und bedarfsgerechte Zufütterung von kohlehydrathaltigen Substraten während einer Hochzelldichte-Fermentation und durch Verwendung eines Gemisches von Glucose, Mannose und Galactose während der Kultivierung eine hohe Ausbeute an gewünschtem Protein, beispielweise EPO, mit hohem Glykosilierungsgrad erhalten wird.

Ein erster Aspekt der Erfindung betrifft somit ein Verfahren zur Erhöhung des Glykosilierungsgrades bei Gewinnung eines glykosilierten Polypeptids aus Säugerzellen, wobei die Säugerzellen in einem geeigneten Medium kultiviert und das gewünschte Polypeptid aus den Zellen oder/und dem Kulturüberstand gewonnen wird,
**dadurch gekennzeichnet,**
dass die Säugerzellen menschliche Zellen sind und dass während der Kultivierung eine kontrollierte und bedarfsgerechte Zugabe von einer Mischung umfassend Glucose, Marinose und Galactose, abhängig vom jeweiligen Bedarf der Zelle erfolgt.

Besonders gute Ergebnisse wurden mit Nährmedien erzielt, die eine Mischung von Glucose, Galactose und Mannose, beispielsweise im Masseverhältnis von 1:(0,5-3):(1-5) und insbesondere von 1:(0,7-2,4):(1,8-4,0) enthalten, wobei die Kohlenhydrate jeweils besonders bevorzugt in der D(+)-Form eingesetzt werden. Die Gesamtkonzentration aller Zucker liegt während der Fermentation vorzugsweise in einem Bereich von 0,1 bis 10 g/l, besonders bevorzugt in einem Bereich von 2 bis 6 g/l Kulturmedium. Die Zugabe der Kohlenhydratmischung erfolgt abhängig vom jeweiligen Bedarf der Zellen wie im folgenden weiter ausgeführt wird.

Eine bevorzugte Ausführungsform betrifftdas erfindungsgemäße Verfahren, das dadurch gekennzeichnet ist, daß während der Kultivierung zusätzlich eine kontrollierte und bedarfsgerechte Zugabe mindestens einer für die jeweils kultivierte Zellinie essentielle Aminosäure, abhängig vom jeweiligen Bedarf der Zellen erfolgt.

Um eine bedarfsgerechte Zugabe von Nährstoffen zu ermöglichen, wird kontinuierlich oder in geeigneten Zeitintervallen, z.B. mindestens einmal täglich, die Konzentration von Parametern bestimmt, die in Korrelation zum Nährstoffbedarf der Zellen stehen und deren Verbrauchsraten ermittelt. Auf diese Weise können die für den Bedarf der Zellen benötigten Nährstoffe quantitativ oder/und qualitativ ermittelt und in entsprechender Zusammensetzung bzw. Menge dem Kulturmedium zugeführt werden. Solche Parameter können Nährstoffe oder Stoffwechselprodukte der Zellen sein, wie etwa die Glutamin-, die Ammonium-, die Glucose- oder/und die Lactatkonzentration, insbesondere die Glutamin-Konzentration.

Aufgrund der kontrollierten und bedarfsgerechten Zugabe von Nährstoffen kann selbst bei einer Hochzelldichtefermention (Zelldichte bei der Ernte > $10 \times 10^5$ Zellen/ml und vorzugsweise > $20 \times 10^5$ Zellen/ml) in Großfermentern (Volumen > 1 1, z.B. 50 bis 10 000 l) eine deutlich verbesserte Glykosilierung erhalten werden.

Die gemäß dieser Ausführungsform der Erfindung zugegebenen Nährstoffe umfassen essentielle Aminosäuren, z.B. Glutamin oder/und Tryptophan und eine Mischung von Glucose, Mannose und Galactose sowie vorzugsweise weiterhin nichtessentielle Aminosäuren, Vitamine, Spurenelemente, Salze oder/und Wachstumsfaktoren, z.B. Insulin. Vorzugsweise enthalten die Nährstoffe mindestens eine essentielle Aminosäure und eine Mischung von Glucose, Mannose und Galactose. Diese Nährstoffe werden vorzugsweise in gelöstem Zustand der Fermentationskultur zudosiert. Die Nährstoffe enthalten vorzugsweise zumindest Glutamin und Kohlenhydrate, wobei eine Mischung von Glucose, Galactose und Mannose eingesetzt wird. Weiterhin ist bevorzugt, daß die Zugabe der Nährstoffe über die gesamte Wachstumsphase der Zellen bedarfsgerecht, d.h. abhängig von der im Kulturmedium gemessenen Konzentration der ausgewählten Parameter erfolgt.

Das Mengenverhältnis von Glutamin zu Kohlenhydraten in der Nährstofflösung wird vorzugsweise so gewählt, daß es dem Verbrauchsverhältnis im Fermenter im wesentlichen entspricht. Auf diese Weise kann eine weitgehend gleichbleibende Konzentration einzelner Substrate im Fermenter erreicht werden. Vorzugsweise wird die Konzentration von Glutamin auf einem Wert gehalten, der < 150 mg/l im Kulturmedium ist und die Entstehung einer Ammoniumkonzentration $\geq 2{,}3$ mmol/l im Kulturmedium verhindert. Die Gesamtkonzentration der Zucker liegt während der Fermentation - wie bereits ausgeführt - vorzugsweise in einem Bereich von 0,1 bis 10 g/l, besonders bevorzugt in einem Bereich von 2 bis 6 g/l Kulturmedium.

Die verwendete Nährstofflösung enthält ein Masseverhältnis von Glutamin zu Zuckern, das vorzugsweise im Bereich von 1:3 bis 20 und besonders bevorzugt von 1:5 bis 15, bezogen auf die gesamten Zucker, liegt. In einer Nährstofflösung, die Glutamin sowie die drei Zucker Glucose, Galactose und Mannose enthält, liegt das Masseverhältnis von Glutamin zu den Zuckern vorzugsweise bei 1:(1 bis 3):(1 bis 5):(2 bis 8) und besonders bevorzugt bei 1:(1,5 bis 2,2):(1,5 bis 3,6):(4 bis 6).

Das erfindungsgemäße Verfahren ist grundsätzlich zur Herstellung beliebiger glykosilierter Polypeptide geeignet. Bevorzugt sind jedoch Polypeptide, die einen oder mehrere Sialinsäurereste tragen, da durch das erfindungsgemäße Verfahren besonders der Sialylierungsgrad der Polypeptide erhöht werden kann. Weiterhin kann auch die Antennärität beeinflußt werden.

Das erfindungsgemäße Verfahren ist besonders zur Herstellung von therapeutisch einsetzbaren Polypeptiden geeignet, da deren biologische Aktivität und somit auch deren pharmazeutische Wirksamkeit von der Glykosilierung und insbesondere vom Sialylierungsgrad oder/und von der Antennärität abhängt. Beispielsweise können die glykosilierten Polypeptide ausgewählt werden aus der Gruppe bestehend aus physiologisch aktiven Glykoproteinen wie etwa Lymphokinen, Cytokinen, Immunglobulinen und Hormonen, z.B. EPO, Thrombopoietin (TPO), G-CSF, GM-CSF, Interleukinen, Interferonen, Blutgerinnungsfaktoren und Gewebsplasminogenaktivatoren. Die Polypeptide können natürliche humane Polypeptide oder rekombinante Muteine solcher humanen Polypeptide sein. Besonders bevorzugt ist das glykosilierte Polypeptid EPO.

Zur Kultivierung der verwendeten Zellen werden humane Zellen verwendet. Weiterhin ist bevorzugt, daß die Zellen kontinuierliche Zellinien humanen Ursprungs sind, wie etwa die humanen Zellinien HeLaS3 (Puck et al., J. Exp. Meth. 103 (1956), 273-284), Namalwa (Nadkarni et al., Cancer 23 (1969), 64-79), HT1080 (Rasheed et al., Cancer 33 (1973), 1027-1033) oder davon abgeleitete Zellinien. Die Produktion des gewünschten Polypeptids in den kultivierten Zellen kann

(a) durch Expression eines natürlichen endogenen Gens,
(b) durch Expression eines aktivierten endogenen Gens oder/und
(c) durch Expression eines exogenen Gens (rekombinant) erfolgen.

Besonders bevorzugt sind Zellen, in der in das gewünschte Polypeptid durch Expression eines durch homologe Rekombination aktivierten endogenen Gens erfolgt, die zur Produktion großer Mengen an EPO in der Lage sind.

Die Kultivierung der Zellen kann grundsätzlich auf beliebige Weise erfolgen. Bevorzugt ist jedoch die Kultivierung als Suspension. Weiterhin ist bevorzugt, daß die Zellen in einem Medium mit geringem Serumgehalt, z.B. maximal 1 % (v/v) oder insbesondere in einem serumfreien Medium, z.B. in einem serumfreien, proteinarmen Fermentationsmedium (vgl. z.B. WO96/35718) kultiviert werden. Beispiele für geeignete Kulturmedien sind Basalmedien wie z.B. RPMI 1640, DMEM, F12 oder eRDF mit entsprechenden Zusätzen. Das erfindungsgemäße Verfahren erlaubt eine Kultivierung in Großfermentern, d.h. in einem Kulturvolumen von mehr als 1 l, vorzugsweise mehr als 10 l, beispielsweise 50 l bis 10.000 l. Weiterhin erlaubt das erfindungsgemäße Verfahren eine Hochzelldichtefermentation, das bedeutet, daß die Konzentration der Zellen nach der Wachstumsphase (d.h. zum Zeitpunkt der Ernte) mehr als $10 \times 10^5$ Zellen/ml und besonders bevorzugt mehr als $20 \times 10^5$ Zellen/ml beträgt.

Die Kultivierung wird vorzugsweise als wiederholt satzweiser Betrieb mit bedarfsgerechter Zufütterung durchgeführt, wobei nach einer Wachstumsphase ein Teil der Kulturbrühe geerntet wird und der Rest der Kulturbrühe im Fermenter bleibt, der anschließend wieder bis zum Arbeitsvolumen mit frischem Medium befüllt wird. Durch das erfindungsgemäße Verfahren kann das gewünschte glykosilierte Polypeptid in sehr hohen Ausbeuten geerntet werden. So beträgt die Konzentration zum Erntezeitpunkt beispielsweise mindestens 30 mg und insbesondere mindestens 40 mg des gewünschten Polypeptids pro l Kulturmedium.

In einer weiteren Ausführungsform ist das Verfahren dadurch gekennzeichnet ist, daß die Kultivierung bei einer Temperatur von $\leq 35{,}5$ °C, vorzugsweise zwischen 33 und 35,0 °C erfolgt. Überraschenderweise wurde nämlich festgestellt, daß durch Temperatursenkung bei der Kultivierung der Anteil an Polypeptiden mit gewünschter Glykosilierung deutlich erhöht wird.

Weiterhin wird die Erfindung durch die nachfolgenden Figuren und Beispiele betreffend die Herstellung von EPO in HeLa S3 Zellen erläutert.

Es zeigen:

Figur 1     den relativen Anteil einzelner EPO-Isoformen in Abhängigkeit von den dem Kulturmedium zugesetzten Kohlenhydraten und

Figur 2     die biologische Aktivität von EPO-Präparaten in Abhängigkeit von den dem Kulturmedium zugesetzten Kohlenhydraten.

**Beispiele**

**Beispiel 1 Bestimmung der spezifischen Aktivität in vivo von EPO**

Für die biologische Aktivität in vivo bei EPO ist der Glykosilierungsgrad, z.B. die Anzahl von tetraantennären Strukturen bezogen auf die Gesamtzahl von Kohlenhydratketten, die Anzahlvon N-Acetyl-Lactosamin-Einheiten bezogen auf eine Glykosilierungsstelle des EPO-Moleküls und der Sialinsäuregehalt pro EPO-Molekül wesentlich. Entsprechend der Anzahl von endständig gebundenen Sialinsäuren treten bis zu 14 Isoformen auf, die sich aufgrund ihrer Ladung z.B. mit der Kapillarzonenenelektrophorese (CZE) auftrennen lassen. Die hochsialidierten sauersten Isoformen 1 bis 5 sind maßgeblich für die biologische in vivo Aktivität von EPO.

Die dosisabhängige Aktivität von EPO auf die Vermehrung und Differenzierung von Erythrozyten-Vorläuferzellen wurde in vivo in Mäusen über den Anstieg der Reticulocyten im Blut nach EPO-Gabe bestimmt.

Hierzu wurden jeweils acht Mäusen verschiedene Dosen der zu analysierenden EPO-Probe und eines EPO-Standards (abgeglichen gegen den EPO-WHO-Standard) parenteral verabreicht. Die Mäuse wurden anschließend unter konstanten definierten Bedingungen gehalten. 4 Tage nach EPO-Gabe wurde den Mäusen Blut entnommen und die Reticulocyten mit Acridinorange angefärbt. Die Bestimmung der Reticulocytenzahl pro 30 000 Erythrocyten erfolgte mikrofluorimetrisch im Durchflußcytometer durch Analyse des Rotfluoreszenz-Histogramms.

Die Berechnung der biologischen Aktivität erfolgte aus den Werten für die Reticulocytenzahlen der Probe und des Standards bei unterschiedlichen Dosen nach dem von Linder beschriebenen Verfahren der paarweisen Gehaltsbestimmung mit parallelen Geraden (Linder, Planen und Auswerten von Versuchen, 3. Auflage, 1969, Birkenhäuser Verlag, Basel).

**Beispiel 2 Bestimmung des Gehalts an Sialinsäureresten**

Die Bestimmung des Sialinsäuregehaltes erfolgte chromatographisch über HPAEC-PAD (High pH Anion Exchange Chromatography mit Pulsed Amperometric Detection) auf einem Dionex System nach enzymatischer Abspaltung der Sialinsäuren mit Neuraminidase aus Arthrobacter ureafaciens (A. ureaf., Boehringer Mannheim).

Ansätze mit je 22 μg EPO aus verschiedenen Präparationen aus CHO- und humanen Zellinien (z.B. HeLa S3) wurden auf eine EPO-Konzentration von 0,2 mg/ml in 5 mM Na-Phosphat-Puffer, pH 7,2 eingestellt. Je eine Hälfte jedes Ansatzes wurde für die genaue Bestimmung der EPO Menge über RP-HPLC verwendet. Zur 2. Hälfte der Ansätze wurde je 5 mM U Neuraminidase von A. ureaf. gegeben und über Nacht (ca. 18 h) bei 37°C inkubiert. Anschließend wurden die Verdauansätze halbiert, 20-fach auf 500 μl mit $H_2O$ verdünnt und 50 μl davon (entspricht ca. 27 pmol EPO) auf das Dionex System aufgetragen. Hierzu wurden folgende Chromatographie-Parameter verwendet:

| Säule | CarboPac PA 100 |
|---|---|
| Fluß | 1,0 ml/min |
| Detektorempfindlichkeit | 300 nA |

| Gradient | t (min) | % Puffer B |
|---|---|---|
| | 0 | 17 |
| | 7 | 17 |
| | 9 | 100 |
| | 12 | 100 |
| | 13 | 0 |
| | 20 | 0 |
| Puffer A | 0,1 M NaOH | |
| Puffer B | 0,1M NaOH; 0,5 M NA-Acetat | |

Die Menge an Sialinsäuren in der aufgetragenen Probe wurde mit Hilfe einer Eichgerade, die aus Werten eines mitgeführten Sialinsäurestandards (Boehringer Mannheim) erhalten wurde, ermittelt. Der Sialinsäuregehalt (mol Sialinsäure/mol EPO) wurde aus dem Ergebnis der Sialinsäurebestimmung (Dionex System) und der Bestimmung der eingesetzten EPO-Menge über RP-HPLC berechnet.

**Beispiel 3 Bestimmung des Anteils an bi-, tri- und tetraantennären Kohlenhydratstrukturen**

Die Analytik der N-gebundenen Kohlenhydratstrukturen erfolgte chromatographisch über HPAEC-PAD auf einem Dionex System. Die asialo Oligosaccharide von EPO-Präparationen aus CHO- und humanen Zellinien (z.B. HeLa S3) wurden durch enzymatische Abspaltung mit N-Glycosidase F (Boehringer Mannheim) und Neuraminidase aus A.ureaf. (Boehringer Mannheim) gewonnen.

Ca. 30 $\mu$g EPO je Ansatz wurden mittels MicroCon-Ultrazentrifugationseinheiten (Amicon, Ausschlußgröße 10 kD) entsalzt und mit 10 mM Na-Phosphat-Puffer, pH 7,2 auf eine Konzentration von 0,3 mg/ml eingestellt. Anschließend wurde jeder Ansatz mit 1 U N-Glycosidase F und 10 mU Neuraminidase versetzt und über Nacht (ca 18 h) bei 37°C inkubiert. Zur Abtrennung des EPO-Polypeptidanteils von den abgespaltenen Oligosacchariden wurden die Ansätze nach der Inkubation durch Ultrafree-Zentrifugationseinheiten (Millipore, Ausschlußgröße 10 kD) zentrifugiert und das Ultrafree-Device zweimal mit 20 $\mu$l H$_2$O nachgewaschen. Die im Durchlauf enthaltenen Oligosaccharide wurden mit H$_2$O auf 150 $\mu$l aufgefüllt und 100 $\mu$l davon auf dem Dionex System analysiert. Hierzu wurden folgende Chromatographie-Parameter verwendet:

| Säule | CarboPac PA 100 |
|---|---|
| Fluß | 1,0 ml/min |
| Detektorempfindlichkeit | 300 nA |

| Gradient | t (min) | % Puffer B |
|---|---|---|
| | 0 | 0 |
| | 2 | 0 |
| | 60 | 10 |
| | 62 | 100 |
| | 67 | 100 |
| | 69 | 0 |
| | 80 | |
| Puffer A | 0,1 M NaOH | |
| Puffer B | 0,1 M NaOH; 0,5 M Na-Acetat | |

Die Identifikation der Peaks in einem Chromatogramm von N-Zuckern des komplexen Typs erfolgte durch Standard-Oligosaccharide (Oxford Glyco Systems) und wurde durch den enzymatischen Verdau der Oligosaccharide von EPO mit dem Enzym Endo-$\beta$-Galactosidase bzw. Fucosidase und anschließender Analytik auf dem Dionex System verifiziert. Die Berechnung der prozentualen Anteile an bi-, tri- und tetraantennären Strukturen erfolgte über die Flächen der Peaks, die die entsprechende N-Zuckerstruktur repräsentieren, bezogen auf die Gesamtpeakfläche (Summe der Peakflächen von bi-, tri- und tetraantennären Strukturen).

**Beispiel 4 Bestimmung des Anteils an N-Acetyl-Lactosamin-Einheiten und des mittleren Anteils an zusätzlichen N-Acetyl-Lactosamin-Einheiten (Repeats)**

Die Gesamtzahl an N-Acetyl-Lactosamin-Einheiten in den N-gebundenen Kohlenhydratstrukturen von EPO (d.h. in den Core-Kohlenhydratstrukturen) wurde aus den Peakflächen der Chromatogramme der Experimente von Beispiel 3 berechnet.

Die Berechnung der Anzahl des mittleren Gehalts (n) von N-Acetyl-Lactosamin-Einheiten pro Kohlenhydratkette war wie folgt:

$$n = \Sigma \ \% \ (bi)x2 + \% \ (tri) \ x3 + \% \ (tetra) \ x4 + \% \ (tri + 1r)x4 + \% \ (tetra + 1r) \ x5 + \% \ (tri + 2r)x5 + \% \ (tetra + 2r)x6 \ \text{wobei}$$

% (bi) =       prozentualer Anteil biantennärer Strukturen bezogen auf die Gesamtzahl von Kohlenhydrat- ketten

% (tri) = prozentualer Anteil triantennärer Strukturen ohne zusätzliche N-Acetyl-Lactosamin-Einheit

% (tetra) = prozentualer Anteil tetraantennärer Strukturen ohne zusätzliche N-Acetyl-Lactosamin-Einheit

% (tri + 1r) = prozentualer Anteil triantennärer Struktu- ren mit 1 zusätzlichen N-Acetyl-Lactosa- min-Einheit

% (tetra + 1r) = prozentualer Anteil tetraantennärer Struk- turen mit 1 zusätzlichen N-Acetyl-Lactosa- min-Einheit

% (tri + 2r) = prozentualer Anteil triantennärer Struktu- ren mit 2 zusätzlichen N-Acetyl-Lactosa- min-Einheiten

% (tetra + 2r) = prozentualer Anteil tetraantennärer Struk- turen mit 2 zusätzlichen N-Acetyl-Lactosa- min-Einheiten.

Ein weiterer wichtiger Parameter ist der Anteil von N-Acetyl-Lactosamin-Einheiten, die als sogenannte Repeats an die Core-Kohlenhydratstrukturen gebunden sein können. Der Repeatgehalt wird als prozentualer Anteil der repeathaltigen Kohlenhydratstrukturen bezogen auf die Gesamtheit der Kohlenhydratstrukturen (bi + tri + tetra = 100%) angegeben. Dieser Anteil von Repeats kann bei EPO-Präparaten aus CHO-Zellen und aus humanen Zellen unterschiedlich sein.

**Beispiel 5: Beeinflussung der biologischen Aktivität von EPO durch kontrollierte und bedarfsgerechte Zufütterung**

Die Kultivierungen wurden als wiederholt satzweiser Betrieb mit bedarfsgerechter Zufütterung (Repeated Fed Batch) bei einer Temperatur von 36,5 °C durchgeführt. Dazu wurde in einem gerührten Fermenter (Gesamtarbeitsvolumen: 10 L) serumfreies proteinarmes Kulturmedium vorgelegt und einmalig mit einer Inokulumskultur beimpft. Die Zelldichte nach Beimpfen lag im Bereich $3 \pm 1 \times 10^5$ lebende Zellen/ml.

Nach einer Wachstumsphase von $144\pm24$ Stunden wurde ein Teil der Kulturbrühe geerntet. Der Rest der Kulturbrühe verblieb im Fermenter und stellte das Inokulum für die nächste Wachstumsphase dar; dazu wurde der Fermenter wieder bis zum Arbeitsvolumen mit frischem Medium aufgefüllt.

Der EPO-haltige Kulturüberstand wurde durch Zentrifugation der Fermentationskultur erhalten.

Während der Wachstumsphase wurde der Kultur kontinuierlich Nährstofflösung zugeführt. Dazu wurde ein Vorratsgefäß mit Nährlösung an den Fermenter gekoppelt. Die Nährstofflösung enthielt Aminosäuren, Vitamine, Insulin, Spurenelemente, Salze, Glutamin und Kohlenhydrate. Es wurden 2 Fermentationen wie folgt durchgeführt:

Bei der Fermentation A enthielt die Nährstofflösung als Zucker D-(+)-Glucose und bei der Fermentation B die Zucker D-(+)-Glucose, D-(+)-Galactose und D-(+)-Mannose. Das Masseverhältnis von Glutamin zu den Zuckern betrug bei Fermentation B 1:2,2:3,6:6. Die Konzentration der einzelnen Zucker in der Nährstofflösung lag zwischen 7,2 und 18 g/l.

Die Glutaminkonzentration in der Kultur wurde bei Fermentation B periodisch analysiert und der Verbrauch berechnet. Der momentane Volumenstrom der Nährstofflösung wurde dem Bedarf der Zellen an Nährstoffen angepaßt. Bei Fermentation A wurde die Glutaminkonzentration nicht als Regelgröße verwendet. Die Nährstofflösung bei der Fermentation B enthielt eine Mischung der Zucker D-(+)Glucose, D-(+)Galactose und D-(+)Mannose im Massenverhältnis von 2:3:5. Während der Kultivierung wurde durch entsprechende Zufütterung die Konzentration aller Zucker im Fermenter in einem Bereich von 2 bis 6 g/l gehalten.

Die Zelldichte veränderte sich durch das Wachstum auf mehr als $20\times10^5$ Zellen/ml, typischerweise $30 \pm 10\times10^5$ Zellen/ml zum Erntezeitpunkt. Die Konzentration an EPO betrug zum Erntezeitpunkt typischerweise $40\pm10$ mg/l.

Aus geernteten Kulturbrühen wurde die Konzentration an humanem Erythropoietin z.B. durch ELISA bestimmt. Eine prozentuale Verteilung der auftretenden Isoformen dieses Proteins wurden z.B. durch Trennung mittels Kapillarzonenelektrophorese (CZE) bestimmt.

Tabelle 1 zeigt den Vergleich der EPO-Isoformenverteilung zwischen einer Fermentation A mit Zufütterung einer Nährstofflösung mit Glucose und einer Fermentation B mit kontrollierter und bedarfsgerechter Zufütterung einer Nährstofflösung mit Glucose, Mannose und Galactose. Der Anteil der EPO-Isoformen bei Fermentation B wurde als prozentualer Anteil der korrespondierenden Isoformen der Fermentation A berechnet. Letztere wurden auf jeweils 100% normiert. Die Daten belegen, daß die erwünschten höher glykosilierten EPO-Isoformen 2-4 während der Fermentation gegenüber der Fermentation A in maßgeblich höheren Anteil vorliegen.

Tabelle 1:

| Isoformbezeichnung in der CZE | 1 | 2 | 3 | 4 | 5 | 6 | 7 | 8 |
|---|---|---|---|---|---|---|---|---|
| | [%] | [%] | [%] | [%] | [%] | [%] | [%] | [%] |
| Fermentation A: Zufütterung mit Glucose | n.b. | 100 | 100 | 100 | 100 | 100 | 100 | 100 |
| Fermentation B: bedarfsgerechte Zufütterung mit Glucose, Mannose und Galactose | n.b. | 136 | 140 | 115 | 102 | 91 | 76 | 55 |

(fortgesetzt)

| Isoformbezeichnung in der CZE | 1 | 2 | 3 | 4 | 5 | 6 | 7 | 8 |
|---|---|---|---|---|---|---|---|---|
| | [%] | [%] | [%] | [%] | [%] | [%] | [%] | [%] |
| n.b.= nicht bestimmbar, da Wert unter Nachweisgrenze | | | | | | | | |

Das bei Zufütterung erhaltene Isoformenmuster war bei vier aufeinanderfolgenden Ernten aus einer Fermentation mit kontrollierter und bedarfsgerechter Zufütterung der Nährstofflösung reproduzierbar.

**Beispiel 6: Beeinflussung der biologischen Aktivität von EPO durch Veränderung der Kultivierungstemperatur**

Die Durchführung erfolgte wie in Beispiel 5 (Fermentation B) beschrieben im Fed-Splitbatch-Verfahren mit kontrollierter und bedarfsgerechter Zufütterung, außer daß die Fermentertemperatur 35,0 °C anstelle von 36,5 °C betrug und die Fermentation im 1000 l Maßstab durchgeführt wurde.

Tabelle 2 zeigt den Vergleich der EPO-Isoformenverteilung zwischen einer Fermentation C bei 36,5 °C und einer Fermentation D bei 35,0 °C jeweils mit kontrollierter Zufütterung einer Nährstofflösung. Der Anteil der EPO-Isoformen bei Fermentation D wurde als prozentualer Anteil der korrespondierenden Isoformen der Fermentation C berechnet. Letztere wurden auf jeweils 100 % normiert. Die Daten belegen, daß die sauren EPO-Isoformen 2 bis 4 durch Absenkung der Temperatur maßgeblich angehoben werden.

Tabelle 2:

| Isoformbezeichnung in der CZE | 1 | 2 | 3 | 4 | 5 | 6 | 7 | 8 |
|---|---|---|---|---|---|---|---|---|
| Relative Isoformenverteilung | [%] | [%] | [%] | [%] | [%] | [%] | [%] | [%] |
| Fermentation C: Temperatur 36,5 °C | n.b. | 100 | 100 | 100 | 100 | 100 | 100 | 100 |
| Fermentation D: Temperatur 35,0 °C | n.b. | 131 | 116 | 110 | 94 | 100 | 88 | 86 |
| n.b.= nicht bestimmbar, da Wert unter Nachweisgrenze | | | | | | | | |

**Beispiel 7: Beeinflussung der biologischen Aktivität von EPO durch Veränderung der Kohlenhydratzusammensetzung im Medium**

Das im folgenden dargelegte Verfahren belegt die Möglichkeit, die Qualität von humanem Erythropoietin durch Veränderung des Kohlenhydratangebotes im Zufütterungsmedium zu verändern.

Es werden zwei Varianten des oben beschriebenen Verfahrens gezeigt (nachfolgend Fermentation E und Fermentation F genannt), die sich durch die Zusammensetzung der verwendeten Medien unterscheiden.

Für beide Ansätze basierte die Rezeptur des Kulturmediums auf modifiziertem eRDF-Medium. Es wurde kein Serum eingesetzt, sondern rekombinantes Insulin (einziger Proteinzusatz) und weitere Supplemente (z.B. Selenit, Putrescin, Hydrocortison, Eisensulfat), die üblicherweise bei serumfreien bzw. proteinfreien Medien eingesetzt werden.

Die Zufütterungs-Nährlösung basiert ebenfalls auf modifiziertem eRDF-Medium, enthält jedoch nicht die Salze $KCl$, $Na_2HPO_4$ und $NaCl$.

Der maßgebliche Unterschied der Fermentationen E und F liegt im Zusatz von verschiedenen Monosacchariden zum Zufütterungsmedium.

Fermentation E:

Es wurde für Fermentation E der üblicherweise eingesetzte Zucker D-(+)-Glucose verwendet. Die Startkonzentration betrug 3 g/l. Durch eine geeignete Zufütterung der giucosehaltigen Nährlösung wurde die Glucosekonzentration in der Kulturbrühe bei 3 ± 0,5 g/l während der gesamten Kultivierung konstant gehalten.

Die Kultivierungsdauer lag typischerweise bei 100 ± 20 h. Die Konzentration an EPO betrug zum Erntezeitpunkt typischerweise 40 ± 10 mg/l.

Fermentation F:

Für die Fermentation F wurden zusätzlich zu D-(+)-Glucose die Zucker D-(+)-Galactose und D-(+)-Mannose im Verhältnis von ca. 1:2:3 für das Zufütterungsmedium eingesetzt. Während der Kultivierung wurde durch entsprechende Zufütterung die Konzentration aller Zucker in einem Bereich zwischen 0,25 g/l und 3,5 g/l gehalten.

Die Kultivierungsdauer lag typischerweise bei 100 ± 20 Stunden. Die Konzentration an EPO betrug zum Erntezeitpunkt typischerweise 40 ± 10 mg/l.

Erythropoietin wurde aus den Kulturüberständen aufgereinigt. Das durchgeführte Aufreinigungsverfahren war so ausgelegt, daß die Verteilung von relevanten Isoformen des Glykoproteins nicht beeinflußt wurde.

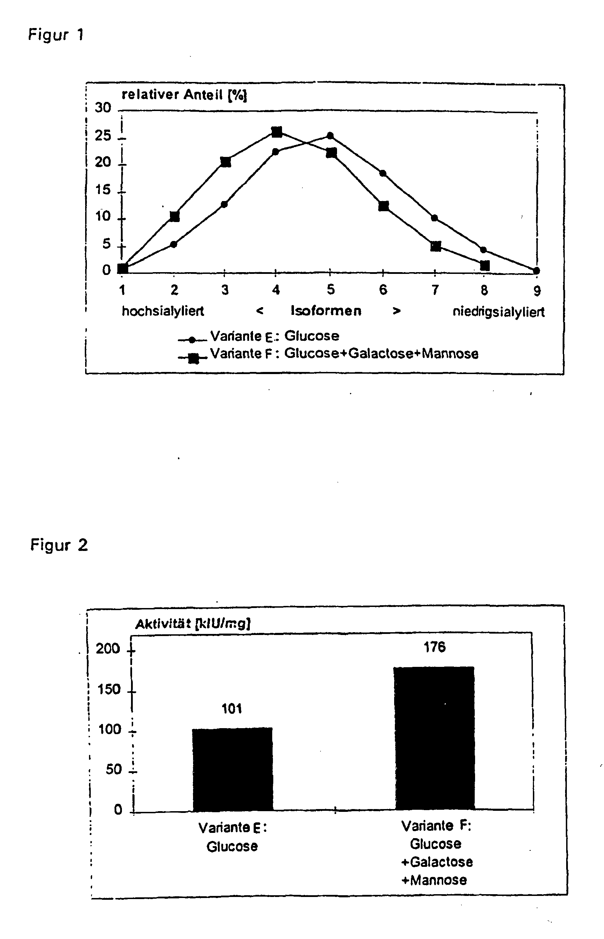

Die Isoformenverteilung des gereinigten Erythropoietin wurde wie zuvor beschrieben bestimmt. Die Kohlenhydratstrukturen der Isoformen von humanem Erythropoietin und ihre Verteilung unterscheiden sich in den geernteten Kulturüberständen von Fermentation E zu Fermentation F. Die Fermentation F zeigt einen deutlich höheren Anteil der Isoformen 2, 3 und 4 gegenüber Fermentation E. Diese Unterschiede werden durch eine Zufütterung der Monosaccharide Mannose und Galactose bewirkt (vgl. Fig. 1).

Die biologische Aktivität, bestimmt durch den Normo-Maus-Test (Beispiel 1), korreliert mit der Verteilung und den Kohlenhydratstrukturen der EPO-Isoformen (Fig. 2). Die Kohlenhydratstrukturen der aus den Kulturüberständen E und F erhaltenen EPO-Präparationen wurden mit CZE- und HPAEC-Analytik untersucht.

In Tabelle 3 sind die Antennärität (Gehalt an Bi- Tri- und Tetrastrukturen), der Gehalt an N-Acetyl-Lactosamineinheiten (LE), der Sialinsäuregehalt (SA) und das Produkt aus LE und SA der beiden EPO-Präparationen dargestellt.

Tabelle 3

|  | bi [%] | tri [%] | tetra [%] | SA-Gehalt | LE-Gehalt | LE×SA |
|---|---|---|---|---|---|---|
|  |  |  |  |  |  |  |
| Fermentation E | 12,6 | 25,4 | 62,0 | 10,8 | 10,8 | 116,7 |
| Fermentation F | 10,1 | 19,2 | 70,6 | 11,6 | 11,25 | 130,5 |

**Patentansprüche**

1. Verfahren zur Erhöhung des Glykosilierungsgrades bei Gewinnung eines glykosilierten Polypeptids aus Säugerzellen, wobei die Säugerzellen in einem geeigneten Medium kultiviert und das gewünschte Polypeptid aus den Zellen oder/und dem Kulturüberstand gewonnen wird,
**dadurch gekennzeichnet,**
**dass** die Säugerzellen menschliche Zellen sind und dass während der Kultivierung eine kontrollierte und bedarfsgerechte Zugabe von einer Mischung umfassend Glucose, Mannose und Galactose, abhängig vom jeweiligen Bedarf der Zelle erfolgt.

2. Verfahren nach Anspruch 1,
**dadurch gekennzeichnet,**
**dass** während der Kultivierung zusätzlich eine kontrollierte und bedarfsgerechte Zugabe mindestens einer essentiellen Aminosäure erfolgt.

3. Verfahren nach Anspruch 1 oder 2,
**dadurch gekennzeichnet,**
**dass** der Bedarf der Zellen über eine Messung der Konzentration von Parametern ausgewählt aus Nährstoffen und Stoffwechselprodukten bestimmt wird.

4. Verfahren nach Anspruch 3,
**dadurch gekennzeichnet,**
**dass** die Glutamin-Konzentration als Regelgröße bestimmt wird.

5. Verfahren nach einem der vorhergehenden Ansprüche,
**dadurch gekennzeichnet,**
**dass** die Nährstoffe weiterhin nichtessentielle Aminosäuren, Vitamine, Spurenelemente, Salze oder/und Wachstumsfaktoren, z.B. Insulin, umfassen.

**6.** Verfahren nach einem der vorhergehenden Ansprüche,
**dadurch gekennzeichnet,**
**dass** das glykosilierte Polypeptid EPO ist.

**7.** Verfahren nach einem der vorhergehenden Ansprüche,
**dadurch gekennzeichnet,**
**dass** die Kultivierung bei einer Temperatur $\leq$ 35,5 °C erfolgt.

**Claims**

**1.** Process for increasing the degree of glycosylation when isolating a glycosylated polypeptide from mammalian cells, wherein the mammalian cells are cultured in a suitable medium and the desired polypeptide is isolated from the cells or/and the culture supernatant,
**characterized in that**
the mammalian cells are human cells and that a mixture comprising glucose, mannose and galactose is added during the culture in a controlled and demand-oriented manner depending on the respective requirement of the cell.

**2.** Process according to claim 1,
**characterized in that**
a controlled and demand-oriented addition of at least one essential amino acid is additionally carried out during the culture.

**3.** Process according to claim 1 or 2,
**characterized in that**
the requirement of the cells is determined by a measurement of the concentration of parameters selected from nutrients and metabolic products.

**4.** Process according to claim 3,
**characterized in that**
the glutamine concentration is determined as a controlled variable.

**5.** Process according to one of the previous claims,
**characterized in that**
the nutrients additionally comprise non-essential amino acids, vitamins, trace elements, salts or/and growth factors e.g. insulin.

**6.** Process according to one of the previous claims,
**characterized in that**
the glycosylated polypeptide is EPO.

**7.** Process according to one of the previous claims,
**characterized in that**
the culture is carried out at a temperature of $\leq$ 35.5°C.

**Revendications**

**1.** Procédé pour augmenter le degré de glycosylation lors de l'obtention d'un polypeptide glycosylé à partir de cellules de mammifère, où les cellules de mammifère sont cultivées dans un milieu approprié et le polypeptide souhaité est obtenu à partir des cellules et/ou du surnageant de culture, **caractérisé en ce que** les cellules de mammifère sont des cellules humaines et **en ce que**, pendant la culture, une addition contrôlée et conforme aux besoins d'un mélange comprenant du glucose, du mannose et du galactose a lieu en fonction des besoins respectifs de la cellule.

**2.** Procédé selon la revendication 1 **caractérisé en ce que**, pendant la culture, une addition contrôlée et conforme aux besoins d'au moins un aminoacide essentiel a lieu en outre.

**3.** Procédé selon la revendication 1 ou 2 **caractérisé en ce que** les besoins des cellules sont déterminés par le biais d'une mesure de la concentration de paramètres choisis parmi les nutriments et les métabolites.

4. Procédé selon la revendication 3 **caractérisé en ce que** la concentration de glutamine est déterminée en tant que grandeur régulée.

5. Procédé selon l'une des revendications précédentes **caractérisé en ce que** les nutriments comprennent en outre des aminoacides non essentiels, des vitamines, des éléments à l'état de traces, des sels et/ou des facteurs de croissance, par exemple l'insuline.

6. Procédé selon l'une des revendications précédentes **caractérisé en ce que** le polypeptide glycosylé est l'EPO.

7. Procédé selon l'une des revendications précédentes **caractérisé en ce que** la culture a lieu à une température ≤ 35,5°C.

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP H6292592 B
- JP H629592 B
- EP 0148605 B
- EP 0205564 B
- EP 0428267 A
- WO 9635718 A

**In der Beschreibung aufgeführte Nicht-Patentliteratur**

- **LJUNGGREN ; HÄGGSTRÖM.** *Biotech. Bioeng.,* 1994, vol. 44, 808-818
- **LJUNGGREN et al.** *Biotech. Lett.,* vol. 12, 705-710
- **MIRABET et al.** *Biotechnol. Bioeng.,* 1997, vol. 56, 530-537
- **GAWLITZEK et al.** *Biotechnol. Bioeng.,* 1998, vol. 57, 518-528
- **BORYS et al.** *Biotechnol. Bioeng.,* 1994, vol. 43, 505-514
- **ANDERSEN ; GOOCHEE.** *Biotechnol. Bioeng.,* 1995, vol. 47, 96-105
- **MIYAKE et al.** *J. Biol. Chem.,* 1977, vol. 252, 5558-5564
- **TSAO et al.** *Biotech. Bioeng.,* 1992, vol. 40, 1190-1196
- **NIETO et al.** *Anal. Commun.,* 1996, vol. 33, 425-427
- **TRAN et al.** *J. Chromatogr.,* 1991, vol. 542, 459-471
- **BIETOT et al.** *J. Chromatogr.,* 1997, vol. 759, 177-184
- **WATSON et al.** *Anal. Biochem.,* 1993, vol. 210, 389-393
- **IMAI et al.** *Eur. J. Biochem.,* 1990, vol. 194, 457-462
- **TAKEUCHI et al.** *Proc. Natl. Acad. Sci. USA,* 1989, vol. 86, 7819-7822
- **FUKUDA et al.** *Blood,* 1989, vol. 73, 84-89
- **MORIMOTO et al.** *Glycoconjugate J.,* 1996, vol. 13, 1093-1120
- **PUCK et al.** *J. Exp. Meth.,* 1956, vol. 103, 273-284
- **NADKARNI et al.** *Cancer,* 1969, vol. 23, 64-79
- **RASHEED et al.** *Cancer,* 1973, vol. 33, 1027-1033
- **LINDER.** Planen und Auswerten von Versuchen. Birkenhäuser Verlag, 1969